# EUROPÄISCHE PATENTSCHRIFT

(11) **EP 1 858 519 B1**
(45) Veröffentlichungstag und Bekanntmachung des Hinweises auf die Patenterteilung: **20.02.2013**
(21) Anmeldenummer: 06723388.2
(22) Anmeldetag: 13.03.2006
(51) Int. Cl.: A61K 31/505, A61P 1/00, A61P 29/00

(54) **ECTOIN UND/ODER HYDROXYECTOIN ZUR PROPHYLAXE UND BEHANDLUNG CHRONISCH ENTZÜNDLICHER DARMERKRANKUNGEN**
ECTOIN AND/OR HYDROXYECTOIN FOR THE PREVENTION AND TREATMENT OF INFLAMMATORY BOWEL DISEASES
ECTOIN ET/U HYDROXYECTOIN POUR LA PRÉVENTION ET LE TRAITEMENT DES MALADIES INFLAMMATOIRES CHRONIQUES INTESTINALES

(30) Priorität: 12.03.2005 DE 102005011442
(43) Veröffentlichungstag der Anmeldung: 28.11.2007
(73) Patentinhaber: Bitop Aktiengesellschaft Für Biotechnische Optimierung, 58453 Witten (DE)
(72) Erfinder: SCHWARZ, Thomas, 68199 Mannheim (DE); LENTZEN, Georg, 46483 Wesel (DE); KRUTMANN, Jean, 41844 Wegberg (DE)
(74) Vertreter: Thiel, Christian
(86) Internationale Anmeldenummer: PCT/EP2006/002286
(87) Internationale Veröffentlichungsnummer: WO 2006/097263

(56) Entgegenhaltungen:
- WO-A-92/00744
- WO-A-97/38686
- WO-A2-01/76572
- DE-A1- 10 006 578
- DATABASE WPI Week 199813 Derwent Publications Ltd., London, GB; AN 1998-140922 XP002409885 & JP 10 017478 A (AJINOMOTO KK) 20. Januar 1998 (1998-01-20)

## Beschreibung

Die Erfindung betrifft die Verwendung von Ectoin und/oder Hydroxyectoin zur Herstellung eines Mittels zur Vorbeugung und Behandlung von chronisch-entzündlichen Darmerkrankungen sowie diese Wirksubstanzen enthaltende Mittel. Die Erfindung betrifft ferner Mittel, die die vorgenannten kompatiblen Solute erzeugende oder anreichernde Mikroorganismen enthalten.

Die belebte Welt umfasst unterschiedlichste Habitate. Darunter fallen auch Biotope, die auf den ersten Blick lebensfeindlich und doch belebt sind. Dazu zählen zum Beispiel Salzseen, heiße Quellen, Kältewüsten, Geysire oder andere Extremstandorte. Allen diesen Habitaten gemein ist, dass sie Lebensbedingungen liefern, denen biologische Makromoleküle und Strukturen ungeschützt nicht standhalten können.

Um zu überleben, haben insbesondere Mikroorganismen dieser Habitate eine Reihe von Schutzmaßnahmen entwickelt, die ihnen ein Überleben ermöglichen. Diese Mikroorganismen werden als extremophile Mikroorganismen bezeichnet.

Einer dieser Schutzfunktionen extremophiler Mikroorganismen besteht in der Herstellung "kompatibler Solute". Diese Stoffklasse ist sehr heterogen, es finden sich neben Kohlehydrat-Derivaten auch cyclische Aminosäuren, organische Phosphor-Verbindungen und ähnliche Substanzen. Zu den typischen kompatiblen Soluten extremophiler Mikroorganismen zählen Ectoin (2-Methyl-4-Carboxy-3,4,5,6-Tetrahydropyrimidin), Hydroxyectoin (2-Methyl-4-carboxy-5-hydroxy-3,4,5,6-Tetrahydropyrimidin), Firoin (Mannosylglycerat), Firoin-A (Mannosylglyceramid), DGP (Di-Glyzerinphosphat), cDPG (cyclisches Diphosphoglycerat), Prolin, Prolinbetain und DIP (Di-myo-Inositol-Phosphat).

Kompatible Solute sind niedermolekulare, hydrophile Verbindungen, die sehr polar und dadurch sehr gut wasserlöslich sind. Durch den Anmelder hergestellte kompatible Solute aus extremophilen Mikroorganismen sind z.B:

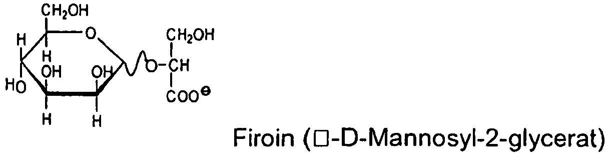

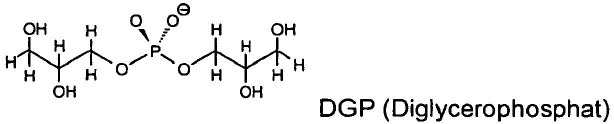

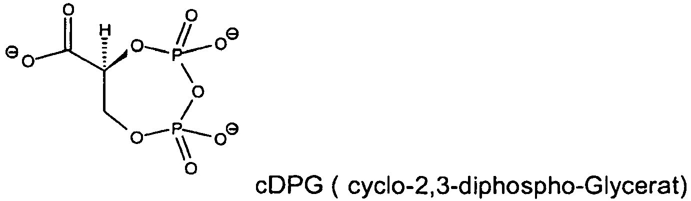

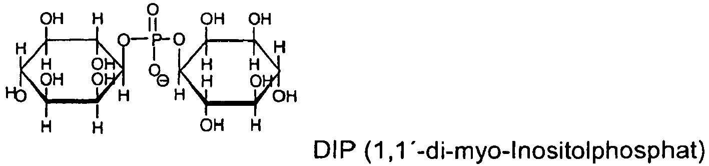

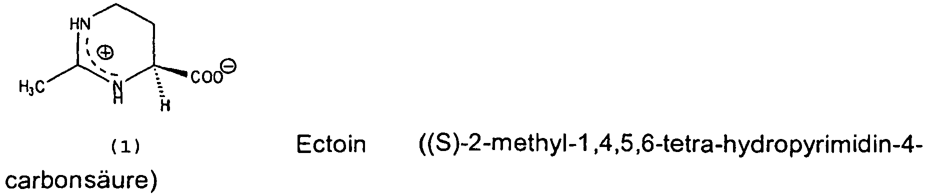

Der Name "kompatible Solute" rührt von der hohen Kompatibilität zum intrazellulären Stoffwechsel von Mikroorganismen und der damit verbundenen sehr hohen Verträglichkeit her. Die Kompatibiltät ist auch bei sehr hohen Konzentrationen im molaren Bereich gegeben. Die Funktion der kompatiblen Solute liegt im Schutz biologischer Strukturen vor zerstörenden Einflüssen.

Diese Funktionen üben die kompatiblen Solute nicht nur bei extremophilen Mikroorganismen, sondern auch bei mesophilen Organismen und auch beim Menschen aus. Dort zeigt sich die Schutzfunktion in einigen schützenden Applikationen. Die kompatiblen Solute finden Einsatz als Hautschutzmittel (z. B. EP 1 315 473 A oder DE 100 44 985 A). Gleichermaßen stabilisieren kompatible Solute die Proteinstruktur, schützen Proteine vor Abbaureaktionen und stabilisieren Lösungen von Proteinen (exemplarisch in EP 0 671 161 A, US 2003157040 A oder auch EP 1 127 141 A, siehe auch Göller & Galinski, J.Molec. Catalysis B: 1999, 7, 37-45).

Das Dokument WO 92/00744 A1 beschreibt die Verwendung von Inositolphosphat zur Behandlung einer Reihe von Erkrankungen, darunter auch Morbus Crohn.

Die deutsche Patentanmeldung DE 100 06 578 A1 zeigt die Verwendung verschiedener kompatibler Solute als Inhibitoren des enzymatischen Abbaus von biologischen Makromolekülen, beispielsweise Proteinen. Entsprechend ist auch eine Verwendung der kompatiblen Solute zur Behandlung von Erkrankungen denkbar, die durch enzymatischen Abbau von Biopolymeren hervorgerufen werden.

Die WO 01/76572 A2 zeigt die Verwendung von kompatiblen Soluten zum Schutz von Organismen, Organen, Geweben und Zellen vor chemischen Radikalen und anderen oxidativ wirkenden Verbindungen.

In der japanischen Patentanmeidung JP 10017478 A wird die Verwendung von Trehalose, u. a. bei der Behandlung von Colitis ulcerosa beschrieben.

Die WO 97/38686 A1 zeigt die Verwendung von kompatiblen Soluten wie myo-Inositol, Taurin und Betain zur Behandlung von Infektionen, Entzündungen und Disfunktionen des Immunsystems.

Eine Vielzahl synthetischer und halb-synthetischer Komponenten, aber auch mikrobiologische Fermentationsprodukte und mikrobiell hergestellte Produkte werden als Mittel zur oralen Verwendung für die Nahrungsergänzung eingesetzt. Einige Beispiele sind:
● Bakterien als Ergänzung zur humanen und
   Tiernahrung GB 2 026 028 A
● Getränke mit Aminosäure-Mischungen CN 1 117 824 A
● Nahrungsergänzung zur Vermeidung von
   Haarverlust US 5 122 369 A
● Nahrungsergänzung mit krebsvorbeugenden
   Eigenschaften CA 2 419 066 A
● Nahrungsergänzung zur Linderung von
   Stresssymptomen EP 1 383 525 A

Dabei werden Produkten aus mesophilen Bakterien und Pilzen wichtige Bedeutungen zuerkannt. Unbekannt ist bisher die Nutzung von Ectoin oder Hydroxyectoin in Mitteln zur oralen Anwendung zur Nahrungsergänzung und zur Prävention und Therapie von Krankheiten des Magen-Darm-Traktes. Schutzwirkungen kompatibler Solute auf zellulärer Ebene erfolgen bereits in geringen Konzentrationen (≤ 1 mM) in Zellkulturen (Bünger & Driller, Skin Pharmacol Physiol 2004, 17, 232-237). Hautschutzeffekte treten allerdings erst in höheren Konzentrationen auf, da hier die abweisende äußere apolare Hornschicht der Haut erst überwunden werden muss und nur eine geringe Resorptionsrate der kompatiblen Solute durch die Haut gegeben ist.

Bei oraler Aufnahme der Ectoine kann eine wesentlich bessere Resorption des Ectoins erwartet sowie ein direkter Schutz der Zellen des Verdauungstraktes erreicht werden.

Diese im folgenden beschriebenen Effekte lassen sich auch spezifisch zur positiven Beeinflussung des Ernährungs- und Gesundheitsstatus von Menschen und Tieren anwenden und damit über die Vermarktung der kompatiblen Solute aus extremophilen Mikroorganismen oder der Biomasse extremophiler Mikroorganismen als Nahrungsergänzungsstoffe und als Arzneimittel gewerblich nutzen.

Dies vorausgeschickt betrifft die Erfindung die Benutzung von Ectoin und Hydroxyectoin in der Prophylaxe und Theraphie sowie als Nahrungsergänzung.

Somit ist ein Gegenstand der Erfindung die eingangs genannte Verwendung von Ectoin und Hydroxyectoin zur Vorbeugung und Behandlung von chronisch-entzündlichen Darmerkrankungen.

Ein weiterer Gegenstand der Erfindung sind Mittel, die Ectoin/Hydroxyectoin zusammen mit Vitaminen, Mineralstoffen und/oder üblichen Hilfsmitteln enthalten. Ein weiterer Gegenstand sind Nahrungsmittel mit einem entsprechenden Gehalt an Ectoin/Hydroxyectoin.

Schließlich ist Gegenstand der Erfindung ein Mittel, das Ectoin oder Hydroxyectoin produzierende oder anreichernde Mikroorganismen enthält.

Bevorzugte Ausführungsformen der Erfindung sind Gegenstand der Unteransprüche.

Überraschenderweise wurde nun gefunden, dass die orale Gabe von Ectoin in der Lage ist, den Verlauf von chronisch-entzündlichen Darmerkrankungen günstig zu beeinflussen. In einem Mausmodell für Morbus Crohn konnte gezeigt werden, dass sich der Schweregrad und die Dauer der Entzündung durch eine orale Substitution der Mäuse mit Ectoin/Hydroxyectoin günstig beeinflussen läßt. Immunologische Untersuchungen zeigten zudem, dass die für chronischentzündliche Darmerkrankungen typische TH-2 TH-1 Antwort durch Ectoin/Hydroxyectoin teilweise supprimierbar ist. Der positive Effekt von Ectoin/Hydroxyectoin ist jedoch nicht nur auf eine Linderung der Symptomatik chronisch-entzündlicher Darmerkrankungen beschränkt, vielmehr zeigte sich hier auch ein ausgeprägtes präventives Potential. So ließ sich die durch bestimmte Nahrungsmittelbestandteile, wie z. B. partikuläre Substanzen, die Nahrungsmitteln als Bleichmittel zugesetzt werden, induzierte Auslösung chronisch-entzündlicher Erkrankungen im Tiermodell signifikant hemmen, wenn die Tiere zuvor in einem präventivem Ansatz oral mit Ectoin/Hydroxyectoin behandelt wurden. Es ist davon auszugehen, dass dieser präventive aber auch der therapeutische Effekt wesentlich auf einer antientzündlichen Wirkung von Ectoin/Hydroxyectoin beruht, deren Ziel zum einen die Darm-Epithel-Zelle ist, zum anderen aber auch die an der Pathogenese ursächlich beteiligten T-Helfer-Lymphozyten.

Weiterhin wurde überraschenderweise gefunden, dass auch in Lebensmitteln, die u.a. aufgrund ihres Herstellprozesses in extremer (z. B. stark salzhaltiger) Umgebung gehandhabt werden und bei deren Herstellung mikrobielle Prozesse eine Rolle spielen, das kompatible Solut Ectoin vorkommt. So konnten gezeigt werden, dass die Oberfläche bestimmter Käsesorten wie Harzer Käse oder Limburger Käse signifikante und gut nachweisbare Konzentrationen an Ectoin aufweisen. Ectoin wurde auch im Inneren der beschrieben Käsesorten nachgewiesen. Dadurch ergibt sich der neue Befund, dass die kompatiblen Solute extremophiler Mikroorganismen, wie z.B. Ectoin, seit Jahrhunderten Bestandteil der Nahrungskette des Menschen sind.

Erfindungsgemäß wird eine orale zu verabreichende Darreichungsform beschrieben, in der Ectoin und Hydroxyectoin, oder Präparationen von Mikroorganismen, die Ectoin/Hydroxyectoin enthalten, insbesondere:
Brevibacterium spec.
Halomonas spec.
Rhodothermus spec.
Pyrococcus spec.
Marinococcus spec.
als Nahrungsergänzungsmittel oder als Arzneimittel zur Behandlung von chronisch-entzündlichen Darmerkrankungen Anwendung finden. Die Darreichungsform kann hierbei, ohne die Erfindung hierdurch einzuschränken, auf Grund der physiko-chemischen Eigenschaften des Ectoins/Hydroxyectoins z.B. eine pulverförmige Mischung, eine Tablette, eine Paste oder eine konzentrierte Lösung, entweder in Form von Extrakten aus Mikroorganismen, lebenden oder abgetöteten Mikroorganismen, Gemischen oder als Reinsubstanz sein.

Es ist auch eine Kombination mit anderen Stoffen möglich, solange diese sich nicht gegenseitig negativ beeinflussen. So können in Mitteln zur oralen Verwendung Ectoin/Hydroxyectoin oder Präparationen aus Mikroorganismen, die Ectoin/Hydroxyectoin enthalten, mit Extrakten aus Kräutern und Früchten wie Isländisch Moos, Teufelskralle, Zimt, Schafgarbe, Sternanis, Salbei, Rosmarin, Piment, Pfefferminze, Oregano, Nelke, Löwenzahn, Liebstöckel, Lavendel, Himbeere, Ingwer, Kamille, Kardamon, Koriander, Kümmel, Curcuma, Basilikum, Bohnenkraut kombiniert werden. Weiterhin können Ectoin/Hydroxyectoin enthaltende Mittel zur oralen Verwendung mit natürlichen Ölen wie Nachtkerzenöl, Lachsöl und anderen Fischölen kombiniert werden. Weitere Stoffe, mit denen die kompatiblen Solute oder Präparationen, die kompatible Solute enthalten, kombiniert werden können, sind Mineralien (Zink, Natrium, Kalium, Calcium, Magnesium, Selen, Chrom, Eisen, Kobalt, Kupfer, Mangan, Silizium, Zink), Vitamine (Vitamin A, Vitamin D, Vitamin E, Vitamin K, Vitamin B1, Vitamin B2, Niacin, Vitamin B6, Pantothensäure, Biotin, Folsäure, Vitamin B12, Vitamin C), L-Carnitin und Isoflavone.

Ectoin/Hydroxyectoin lässt sich auch mit festen Nahrungsmitteln kombinieren. Dies gilt insbesondere, z.B. wenn die Solute als hochkonzentrierte wässrige Lösung beigemischt und anschließend getrocknet werden. Wegen der thermischen Stabilität ist dabei auch die Verwendung in Gebäck möglich.

Die Konzentration des Ectoins/Hydroxyectoins liegt dabei im Bereich von 0,01-50 %. Die tägliche Dosis liegt üblicherweise bei 1 bis 2.000 mg/kg Körpergewicht und kann in Abhängigkeit von der Anwendung in weiten Bereichen variieren.

Die Mittel, die Ectoin/Hydroxyectoin enthalten, können Tabletten, Dragees, Kapseln, Pulver, Granulate, Pastillen, wässrige Lösungen, Flüssigampullen und Zäpfchen sein. Die Mittel können auch Mikroorganismen, sowie Extrakte und Präparationen von Mikroorganismen darstellen, die Ectoin/Hydroxyectoin enthalten. Insbesondere können die Mittel auch Extrakte aus Lebensmitteln sowie Extrakte und Präparationen von Mikroorganismen, die in Lebensmitteln vorkommen, enthalten. Die Menge an Ectoin/Hydroxyectoin beträgt dabei wenigstens 100 mg Wirksubstanz pro Dosiseinheit (Verabreichungseinheit), zweckmäßigerweise 200 bis 500 mg.

Kompatible Solute zur oralen Verwendung wie Ectoin, Hydroxyectoin, Mannosylglycerat, Mannosylglyceramid, Di-Glyzerinphosphat, cyclisches Diphosphoglycerat, Prolin, Prolinbetain und Di-myo-Inositol-Phosphat können aus Mikroorganismen und anderen biologischen Quellen gewonnen werden. Bei den Mikroorganismen kann es sich um Mitglieder der Gattung Brevibacterium, Vibrio, Bacillus, Halomonas, Planococcus, Sporosarcina und Marinococcus handeln. Besonders bevorzugt ist die Verwendung von Mikroorganismen, die Ectoin und Hydroxyectoin synthetisieren und in Lebensmitteln vorkommen wie Brevibacterium linens, Brevibacterium casei, Vibrio costicola, Halomonas elongata. Die Substanzen können durch Behandlung der Zellen mit einer Waschlösung gewonnen werden, wobei diese aus Wasser, flüchtigen Stoffen und/oder auf Zellen stabilisierend wirkenden Stoffen besteht. Das Mittel zur oralen Verwendung kann entweder aus gewaschenen, getrockneten Zellen hergestellt werden oder aus den Zellen durch osmotischen Schock isoliert werden.

Alternativ kann Ectoin/Hydroxyectoin durch chemische Synthese erzeugt werden.

Erfindungsgemäß können Ectoin/Hydroxyectoin und Zubereitungen aus extremophilen Mikroorganismen, die Ectoin/Hydroxyectoin enthalten, Verwendung als Nahrungsergänzungsmittel, in diätetischen Lebensmitteln oder ergänzenden bilanzierten Diäten sowie zur Prävention und Behandlung von chronisch-entzündlichen Darmerkrankungen wie z.B. Morbus Crohn finden.

Weitere Ausführungsformen der Erfindung sind die Verwendung von Extrakten und Konzentraten aus Lebensmitteln, die Ectoin/Hydroxyectoin enthalten.

In jenem Fall beruht die Erfindung darauf, dass Ectoin/Hydroxyectoin geeignet ist, als Wirksubstanz zahlreiche Körperfunktionen und Stoffwechselvorgänge positiv zu beeinflussen, insbesondere was den Magen-Darm-Trakt anbetrifft. Insoweit ist Gegenstand der Erfindung die Verwendung dieser Wirksubstanzen als Nahrungsergänzungsmittel und in der medizinischen Prophylaxe und Therapie.

## Patentansprüche

1. Mittel, enthaltend Ectoin und/oder Hydroxyectoin, zur Verwendung in Vorbeugung und Behandlung von chronisch-entzündlichen Darmerkrankungen.

2. Mittel zur Verwendung nach Anspruch 1, **dadurch gekennzeichnet, dass** die Darmerkrankung Morbus Crohn ist.

3. Mittel zur Verwendung nach Anspruch 1 oder 2, **dadurch gekennzeichnet, dass** das Mittel ein Mittel zur oralen Verabreichung ist.

4. Mittel zur Verwendung nach einem der Ansprüche 1 bis 3, **dadurch gekennzeichnet, dass** die Menge an Wirksubstanz wenigstens 100 mg beträgt.

5. Mittel zur Verwendung nach Anspruch 4, **dadurch gekennzeichnet, dass** die Menge an Wirksubstanz 200 bis 500 mg pro Dosiseinheit beträgt.

6. Mittel zur Verwendung nach einem der Ansprüche 1 bis 5, **dadurch gekennzeichnet, dass** das Mittel Vitamine, Mineralstoffe und/oder übliche Hilfsmittel enthält.

7. Mittel zur Verwendung nach einem der Ansprüche 1 bis 6, **dadurch gekennzeichnet, dass** das Mittel ein Nahrungsergänzungsmittel ist.

8. Mittel zur Verwendung nach einem der Ansprüche 1 bis 7, **dadurch gekennzeichnet, dass** das Mittel als Nahrungsmittel vorliegt, das Ectoin und/oder Hydroxyectoin in einer Menge von 0,01 bis 1 Gew.-% enthält.

9. Mittel zur Verwendung nach einem der Ansprüche 1 bis 8, **dadurch gekennzeichnet, dass** das Mittel in Form von Ectoin und/oder Hydroxyectoin enthaltenden Mikroorganismen oder Extrakten oder Präparationen von Mikroorganismen vorliegt.

10. Mittel zur Verwendung nach Anspruch 9, **dadurch gekennzeichnet, dass** es den Mikroorganismus in getrockneter oder gefriergetrockneter Form enthält.

11. Mittel zur Verwendung nach Anspruch 9 oder 10, **dadurch gekennzeichnet, dass** es den Mikroorganismus in lebensfähiger Form enthält.

12. Mittel zur Verwendung nach einem der Ansprüche 9 bis 11 in Pulver-, Tabletten- oder Kapselform.

13. Verwendung von Ectoin und/oder Hydroxyectoin zur Herstellung eines Mittels zur Vorbeugung und Behandlung von chronisch-entzündlichen Darmerkrankungen.

14. Verwendung nach Anspruch 13, **dadurch gekennzeichnet, dass** die Darmerkrankung Morbus Crohn ist.

## Claims

1. Agent containing ectoine and/or hydroxyectoine for use in prevention and treatment of chronically inflammable intestinal diseases.

2. Agent for use according to claim 1, **characterized in that** the intestinal disease is Crohn's disease.

3. Agent for use according to claim 1 or 2, **characterized in that** the agent is an agent for oral application.

4. Agent for use according to any of claims 1 to 3, **characterized in that** the amount of active substance is at least 100 mg.

5. Agent for use according to claim 4, **characterized in that** the amount of active substance is 200 to 500 mg per dosage unit.

6. Agent for use according to any of claims 1 to 5, **characterized in that** the agent comprises vitamins, mineral nutrients and/or customary additives.

7. Agent for use according to any of claims 1 to 6, **characterized in that** the agent is a dietary supplement.

8. Agent for use according to any of claims 1 to 7, **characterized in that** the agent is a foodstuff containing ectoine and/or hydroxyectoine in an amount ranging between 0.01 and 1 % (w/w).

9. Agent for use according to any of claims 1 to 8, **characterized in that** the agent is in form of microorganisms or extracts or preparations of microorganisms containing ectoine and/or hydroxyectoine.

10. Agent for use according to claim 9, **characterized in that** it contains the microorganism in dried or freeze-dried form.

11. Agent for use according to claim 9 or 10, **characterized in that** it contains the microorganism in a viable form.

12. Agent for use according to any of claims 9 to 11 in the form of powder, tablets or capsules.

13. Use of ectoine and/or hydroxyectoine for the production of an agent aimed at preventing and treating chronically inflammable intestinal diseases.

14. Use according to claim 13, **characterized in that** the intestinal disease is Crohn's disease.

## Revendications

1. Produit contenant de l'ectoïne et/ou de l'hydroxyectoïne, pour utilisation dans la prévention et le traitement de maladies intestinales inflammatoires chroniques.

2. Produit pour utilisation selon la revendication 1, **caractérisé en ce que** la maladie intestinale est la maladie de Crohn.

3. Produit pour utilisation selon la revendication 1 ou 2, **caractérisé en ce que** le produit est un produit pour administration orale.

4. Produit pour utilisation selon l'une des revendications 1 à 3, **caractérisé en ce que** la quantité de substance active est d'au moins 100 mg.

5. Produit pour utilisation selon la revendication 4, **caractérisé en ce que** la quantité de substance active est de 200 à 500 mg par dose unitaire.

6. Produit pour utilisation selon l'une des revendications 1 à 5, **caractérisé en ce que** le produit contient des vitamines, des substances minérales et/ou des adjuvants usuels.

7. Produit pour utilisation selon l'une des revendications 1 à 6, **caractérisé en ce que** le produit est un complément alimentaire.

8. Produit pour utilisation selon l'une des revendications 1 à 7, **caractérisé en ce que** le produit se présente sous forme d'aliment qui contient de l'ectoïne et/ou de l'hydroxyectoïne en une quantité de 0,01 à 1 % en poids.

9. Produit pour utilisation selon l'une des revendications 1 à 8, **caractérisé en ce que** le produit se présente sous la forme de microorganismes ou d'extraits ou de préparations de microorganismes contenant de l'ectoïne et/ou de l'hydroxyectoïne.

10. Produit pour utilisation selon la revendication 9, **caractérisé en ce qu'**il contient le microorganisme sous forme séchée ou lyophilisée.

11. Produit pour utilisation selon la revendication 9 ou 10, **caractérisé en ce qu'**il contient le microorganisme sous forme viable.

12. Produit pour utilisation selon l'une des revendications 9 à 11, sous forme de poudre, de comprimés ou de capsules.

13. Utilisation d'ectoïne et/ou d'hydroxyectoïne pour la préparation d'un produit pour la prévention et le traitement de maladies intestinales inflammatoires chroniques.

14. Utilisation selon la revendication 13, **caractérisé en ce que** la maladie intestinale est la maladie de Crohn.
